(19) Europäisches Patentamt
European Patent Office
Office européen des brevets

(11) Numéro de publication: **0 245 417 B1**

(12) # FASCICULE DE BREVET EUROPEEN

(45) Date de publication de fascicule du brevet: **04.03.92**

(51) Int. Cl.⁵: **C07C 255/32**, C07C 255/26, C07C 227/26, C07C 225/02, C07D 209/18, C07F 5/06

(21) Numéro de dépôt: **86906847.8**

(22) Date de dépôt: **19.11.86**

(86) Numéro de dépôt internationale :
**PCT/FR86/00391**

(87) Numéro de publication internationale :
**WO 87/02980 (21.05.87 87/11)**

(54) **NOUVEAU PROCEDE D'OBTENTION D'ALPHA-AMINONITRILES ET LEURS APPLICATIONS EN SYNTHESE ORGANIOUE.**

(30) Priorité: **19.11.85 FR 8517214**

(43) Date de publication de la demande:
**19.11.87 Bulletin 87/47**

(45) Mention de la délivrance du brevet:
**04.03.92 Bulletin 92/10**

(84) Etats contractants désignés:
**DE FR GB IT**

(56) Documents cités:
**FR-A- 2 174 186**
**FR-A- 2 380 256**
**US-A- 3 422 132**
**US-A- 3 883 550**
**US-A- 4 517 130**

(73) Titulaire: **Albert ROLLAND S.A. Société dite**
**49, Rue St-André-des-Arts**
**F-75006 Paris(FR)**

(72) Inventeur: **MOINET, Gérard**
**15, rue Lamartine**
**F-91400 Orsay(FR)**
Inventeur: **IMBERT, Thierry**
**8, La Levrière**
**F-78590 Noisy-Le-Roi(FR)**

(74) Mandataire: **Burtin, Jean-François**
**Cabinet GEFIB 55 Rue Boissonade**
**F-75014 Paris(FR)**

EP 0 245 417 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention se rapporte à la synthèse de nitriles porteurs d'une fonction amine sur le carbone adjacent.

Elle concerne plus particulièrement un procédé de préparation d'$\alpha$-aminonitriles, qui consiste à transformer un nitrile en son homologue supérieur portant une fonction amine en $\alpha$, en une seule opération.

La présente invention a donc plus précisément pour objet le procédé d'obtention d'$\alpha$-aminonitriles de formule générale I :

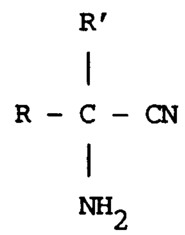

(I)

dans laquelle
R représente un radical alphatique, aromatique ou cyclanique et R' représente un atome d'hydrogène, un radical alcoyle ou alcoyle substitué ou un radical aryle
caractérisé en ce que l'on soumet un nitrile aliphatique, aromatique ou cyclanique, de formule générale II

R- C≡N (II)

dans laquelle R a la définition énoncée ci-dessus,
à l'action d'un agent réducteur métallique choisi dans le groupe constitué par un hydrure d'Aluminium, un sel organomagnesien de formule R'MgX, un sel organozincique de formule R'Zn Hal, un sel organocadmien et un sel organocuprique pour former une imine métallique de formule générale III

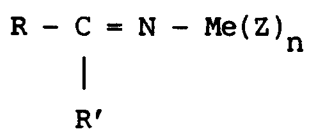

(III)

dans laquelle
R et R' sont tels que précédement définis
Me est un atome de métal choisi parmi l'Aluminium, le Magnésium, le Zinc, le Cadmium et le Cuivre
Z est un radical alcoyle inférieur ou un atome d'halogène
et n est un nombre entier égal à la valence du métal diminué de un
et l'on soumet celle-ci à l'action d'un agent de cyanuration choisi dans le groupe constitué par le cyanure de trimethylsilyle, le cyanure de diéthylaluminium, le cyanure de tributylstannane, les cyanoborohydrures de métal alcalin, les cyanoborohydrures de métal alcalino-terreux, les cyanures de métal alcalin et l'acide cyanhydrique pour obtenir un $\alpha$-amino nitrile de formule générale I.

D'une manière préférée, l'agent réducteur métallique est un hydrure d'Aluminium ayant au moins un atome d'hydrogène libre. L'agent réducteur métallique peut être également un agent réducteur alcoylant ou arylant, comme un sel organomagnésien de formule R'MgX dans laquelle R' est un radical alcoyle substitué ou non, ou un radical aryle ou un sel organizincique de formule HalZnR'
dans laquelle Hal représente un halogène autre que le fluor
et R' possède les significations fournies précédemment ou un sel organocadmien ou un sel organocuprique.

L'agent préféré est l'hydrure de diisobutylaluminium (DIBAL), l'hydrure de diterbutoxyaluminium ou le di(méthoxyéthoxy) aluminohydrure de sodium.

L'agent de cyanuration est choisi dans le groupe constitué par le cyanure de triméthylsilyle, le cyanure de diéthylaluminium, le cyanure de tributylstannane [$(Bu)_3Sn$-CN], les cyanoborohydrures de métal alcalin

ou alcalino-terreux, les cyanures de métal alcalin en présence d'un éther couronne ("Crown Ether"), ou l'acide cyanhydrique en solution dans un solvant oxygéné, comme par exemple le tétrahydrofuranne.

Le tableau suivant fournit une comparaison entre les différents agents réducteurs et les différents agents de cyanuration.

COMPARAISON DES RENDEMENTS OBTENUS PAR HYDROCYANATION DE NITRILES

| Agents de cyanation / Nitriles | $(C_2H_5)_2AlCN$ | $(CH_3)_3SiCN$ | $(C_4H_9)_3SnCn$ | HCN/THF | LiCN/DMF | NaCN/DMF | α-aminonitriles |
|---|---|---|---|---|---|---|---|
| 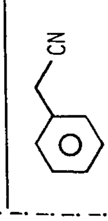 | 65 | 55 / 0 | 24 | 70 | 60 | 60 | 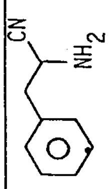 |
| 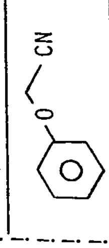 | 22 | 27 / 20 | | | | | 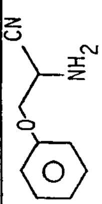 |
| * 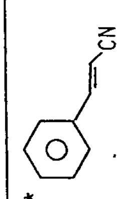 | 20 | 33 | | | | | 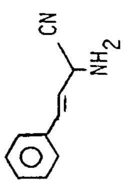 |
| * 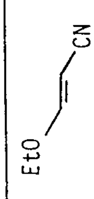 | | 20 | | | | | 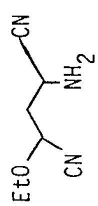 |
| 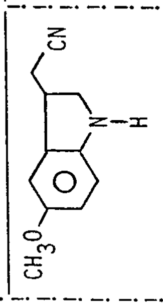 | | 70 | | 70 | | 50 | 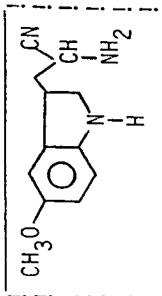 |
| 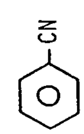 | | 62 / 57 | | | | | 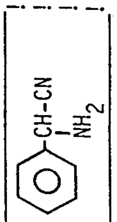 |

A / B

A : Rendement exprimé en % avec l'hydrure de diisobutylaluminium (DiBAH)

B : Rendement exprimé en % avec le dihydro bis(méthoxy-2-éthoxy) aluminate de sodium (VITRIDE)

* : Réaction effectuée à -60°

Sans que ceci ne limite l'invention, le procédé selon l'invention peut être illustré par les deux schémas réactionnels suivants:

1. dans le cas d'un hydrure d'Aluminium comme agent réducteur métallique

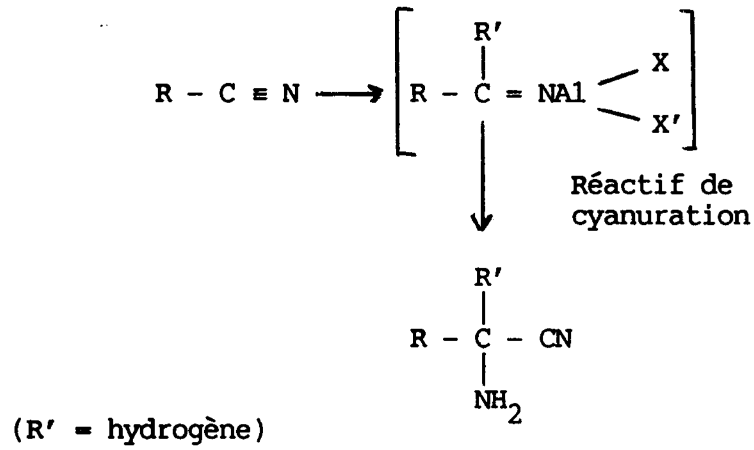


2. dans le cas d'un halogénure organométallique comme réducteur alcoylant

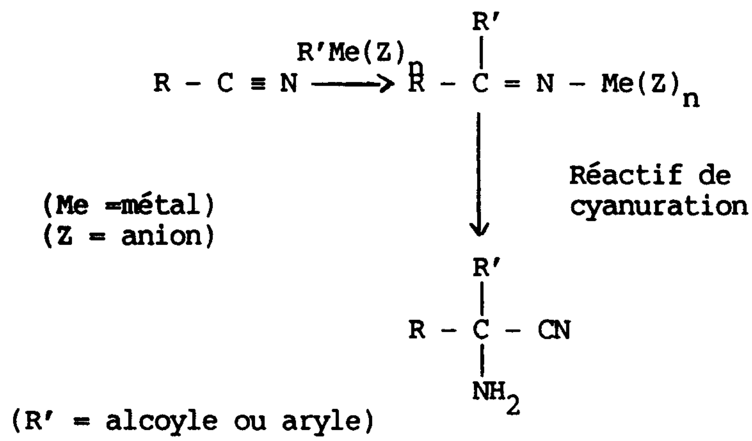


Dans ces deux schémas, R est un radical alcoyle substitué ou non substitué ayant 1 à 30 atomes de carbone; un radical aryle mono- ou bicyclique, substitué ou non substitué, un radical alcényle ayant de 2 à 30 atomes de carbone; un radical hétéroaryle mono- ou bicyclique; un radical aryl-alcoyle dans lequel le radical alcoyle a de 1 à 15 atomes de carbone et où le radical aryle peut porter de 1 à 3 substituants; un radical aryloxy-alcoyle inférieur éventuellement substitué par un à trois substituants sur le radical aryle ou un radical cycloalcényle, ayant de 4 à 7 atomes de carbone alcoyle inférieur.

Pour autant que l'invention soit concernée, le terme alcoyle inférieur désigne un radical hydrocarboné ayant de 1 à 6 atomes de carbone en chaine linéaire ou ramifiée. Des exemples de ceux-ci sont butyle, terbutyle, néopentyle, n-héxyle, méthyle ou éthyle.

Lorsque le radical alcoyle est substitué, il peut porter un groupement fonctionnel vis-à-vis des organométalliques comme un halogène, un radical aminé comme une chaine de la forme :

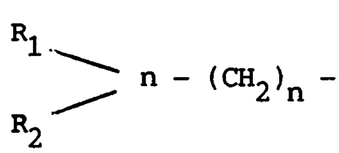

dans laquelle R1 et R2 sont de l'hydrogène, un radical alcoyle, un radical aryle ou une chaine alcoylène et n est un nombre entier variant de 1 à 30.

Il peut porter également un substituant hydroxy, alcoxy ou aryloxy pour former une chaine de la forme :

$R_1O - (CH_2)_n -$

dans laquelle $R_1$ est de l'hydrogène, un radical alcoyle ou un radical aryle et n est défini comme ci-dessus.

Il peut porter aussi un substituant carbonyle ou alcoxycarbonyle préalablement protégé sous forme de dioxolane ou d'ortho-ester selon la

formule

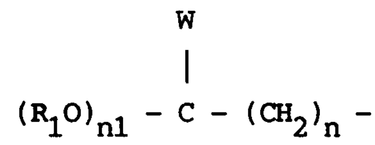

dans laquelle n 1 est égal à 1 ou 2
et W est un radical alcoyle, de l'oxygène ou un alcoxy inférieur
Un radical alcényle inférieur est un radical hydrocarboné portant une double liaison, comportant de 2 à 6 atomes de carbone. On pourra citer, à titre d'exemple de radicaux alcényles, le radical allyle, le radical méthallyle, le radical but-2-ényle, le radical isopropényle ou le radical méthyl-3-butylényle.

Lorsque R représente un radical aryle substitué, les substituants peuvent être de 1 à 3 atomes d'halogène, un radical trifluorométhyle ou trifluorométhoxy, 1 à 3 radicaux alcoyle inférieur ou alcoxy inférieur.

Un radical aralcoyle inférieur est un radical arylmonocyclique porteur d'une chaine hydrocarbonée ayant de 1 à 6 atomes de carbone en chaine droite ou ramifiée. Des exemples de tels radicaux sont le radical benzyle, phényl-éthyle, α-méthylphényl-éthyle, 2,6-dichlorobenzyle ou 2,3,5-triméthoxyl-benzyle. Un radical (hétéroaryle)alcoyle inférieur est un radical hétérocyclique aromatique porteur d'une chaine hydrocarbonée ayant de 1 à 6 atomes de carbone. Des exemples de tels radicaux sont par exemple le (pyridyl-2)méthyle ou le furyl-éthyle, le pyranyl-éthyle ou le (thiényl-2)méthyle, indolylméthyle.

Un radical hétéroaryle désigne une structure cyclique, saturée ou insaturée, comportant de 4 à 7 chainons comme l'azétidine, la pyrrolidine, la pipéridine ou l'hexaméthylène-imine. Lorsque cette chaine est interrompue par un ou deux hétéro-atomes tels que le soufre, l'oxygène et/ou un groupe imino, le cycle qui en résulte est par exemple une tétrahydropyrimidine, une tétrahydro-oxazine, une morpholine, une thiazine, une pyrazolidine ou une pipérazine. Ces cycles peuvent porter des substituants comme par exemple des radicaux alcoyle inférieur, hydroxy-alcoyle inférieur, pyridyle, phényle non substitué ou substitue ou pyrimidyle.

L'invention peut encore être définie par les modes d'exécution suivants qui sont actuellement préférés :

1° - la réaction d'imination est effectuée dans un solvant inerte comme un carbure aromatique, par exemple le toluène ou le xylène ou un éther linéaire cyclique, par exemple le tétrahydrofuranne ou le dioxanne;

2° - la réaction d'imination est effectuée à basse température, plus particulièrement entre -30° et +10°;

3° - réaction de cyanuration est effectuée dans le même solvant que celui de la réaction d'imination;

4° - la réaction de cyanuration est effectuée en l'absence ou en présence d'un acide de Lewis comme par exemple le chlorure d'aluminium, le chlorure de zinc, le chlorure stannique ou le trifluorure de bore;

5° - la réaction de cyanuration est effectuée dans le mélange réactionnel sans isolement préalable de l'imine;

6° - la réaction de cyanuration est effectuée à une température comprise entre -20°C et +50°C, de préférence au voisinage de 0°.

Ainsi qu'il en ressort, le procédé selon l'invention se caractérise par sa grande simplicité de mise en oeuvre. Dans un premier temps, on fait réagir à basse température et sous atmosphère inerte le nitrile RCN en solution dans un solvant inerte avec l'agent de réduction métallique, puis après un bref contact, on ajoute sous agitation le réactif de cyanuration. Après les purifications usuelles, on obtient l'α-aminonitrile.

Le procédé selon l'invention trouve un emploi dans la préparation des matières premières de formule générale IV :

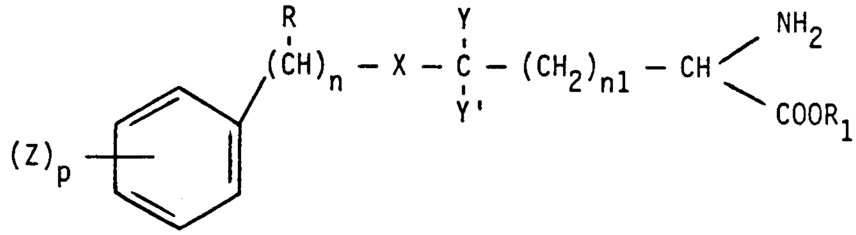

(IV)

comme décrites dans la demande de brevet européen.85. 401360. 4 du 4 juillet 1985

De tels amino-acides ou amino-esters sont obtenus au départ des α-cyanoamines de formule générale I, soit par hydrolyse acide dans un acide minéral dilué, soit par hydrolyse enzymatique, soit encore par une réaction de Pinner fournissant d'abord un imidate qui conduit à un α-aminoester:

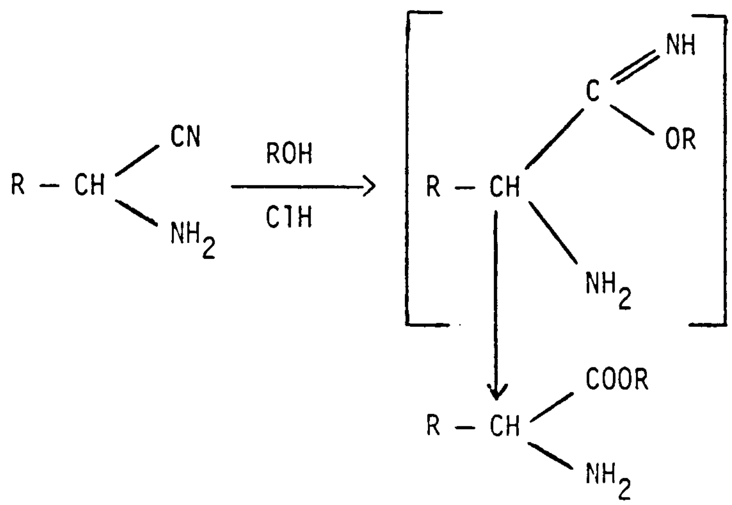


Il est également possible d'accéder, selon le procédé de l'invention, à des α-cyano-amines α-substituées qui constituent des précurseurs précieux pour la synthèse d'α-aminoacides α-substitués comme par exemple l'α-méthyl DOPA ou les α-fluoro-amino-acides dénommés enzymes-suicide.

Le schéma de synthèse est le suivant:

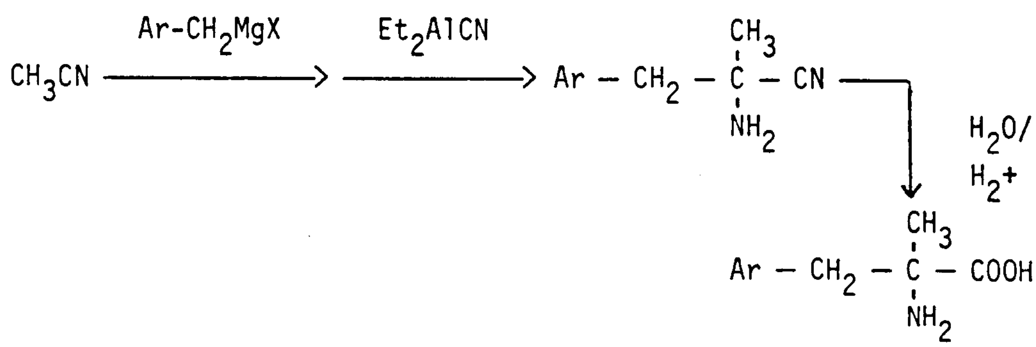

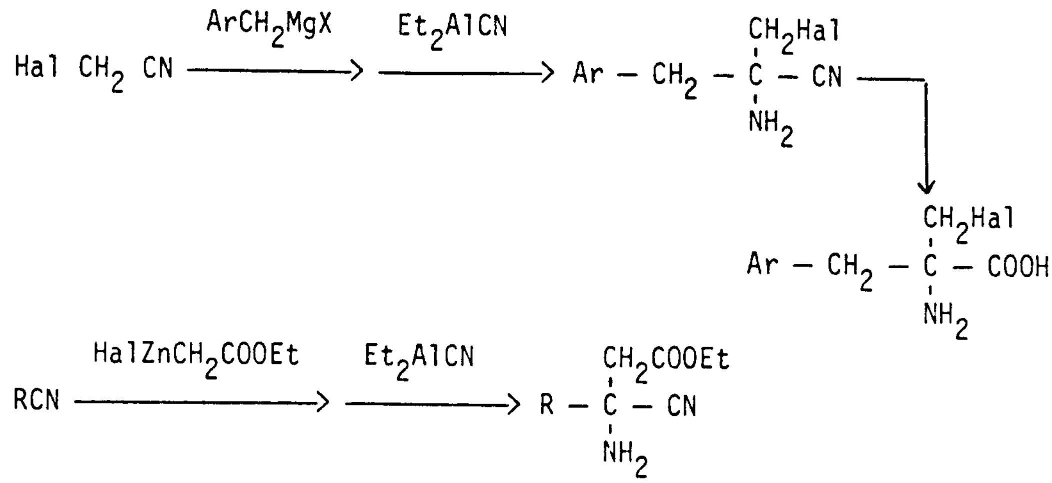


Un autre intérêt du procédé selon l'invention réside dans la possibilité d'obtenir une très grande variété d'intermédiaires de synthèse par attaque du groupe nitrile:

- hydrolyse en amino-acide,
- aminolyse en amidine,
- hydrazinolyse en amidrazone,
- attaque par l'hydroxylamine en amidoxime de formule:

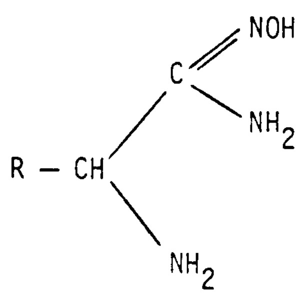

Tous ces composés sont des intermédiaires précieux pour former après cyclisation des composés hétérocycliques tels qu'oxazolines, imidazolines, oxadiazoles ou triazoles ...

Un autre intérêt du procédé selon l'invention réside dans le fait que les α-cyano-amines de formule générale I peuvent être soumises à une hydrolyse enzymatique pour former préférentiellement un amino-acide optiquement actif. On a donc ainsi la possibilité d'accéder avec un bon rendement soit à un amino-acide de configuration antipodale comme par exemple la D-alanine qui trouve de nombreuses utilisations dans la synthèse des Enkephalines de produits antibactériens ou d'inhibiteurs enzymatiques soit à un amino de configuration naturelle.

Il est possible d'accéder par le procédé de l'invention à des amino-acides ou à des aminonitriles aromatiques ou hétérocycliques, substitués ou non substitués, dont l'isolement dans la nature est difficile ou peu rentable. On peut ainsi obtenir au départ de la 2-cyano-pyridine le (pyridyl-2)-2-cyano-2-aminométhane que l'on hydrolyse en acide 2(pyridyl-2)-2-amino-acétique.

On peut également obtenir par le procédé selon l'invention la phénylglycine ou la p-hydroxyphénylglycine qui sont des matières premières précieuses pour la fabrication de pénicillines semi-synthétiques.

Les exemples suivants décrivent et illustrent l'invention. Ils ne la limitent en aucune façon.

<u>EXEMPLE 1</u>:

<u>Préparation de l'amino-2-phénoxy-3-propionitrile</u>

A) <u>Première méthode</u>

a) 5 millimoles (0,665 g) de phénoxyacétonitrile sont mises en solution dans 8 ml de toluène anhydre à 0° C, sous atmosphère d'argon. 7,5 millimoles d'une solution toluénique 1,5M d'hydrure de diisobutylaluminium (5 ml) sont ajoutées, goutte à goutte, à cette température. L'agitation est maintenue pendant 1 heure à 0° C puis 5,9 ml (1,3 éq.) de cyanure de diéthylaluminium sont ajoutés. Le milieu réactionnel est agité pendant 3 heures à température ambiante puis hydrolysé par 4 ml de méthanol et par du sulfate de sodium en bouillie. Après purification par chromatographie liquide sur silice, on obtient 0,182 g d'amino-2-phénoxy-3-propionitrile, sous forme d'un solide blanc crème; P.F.: 65° C; Rendement: 22%.

b) En opérant de la même façon mais en remplaçant le cyanure de diéthylaluminium par le cyanure de triméthylsilyle, on obtient 0,220 g d'amino-2-phénoxy-3-propionitrile; P.f.: 65° C; Rendement: 27%.

c) En opérant selon la même méthode, on a également préparé:

- le phényl-4-amino-2-butyronitrile, à partir du phényl-3-propionitrile;
- l'amino-2-capronitrile, à partir du valéronitrile;
- l'amino-2-(cyclohex-1-enyl)-3-propionitrile, à partir du (cyclohex-1-enyl)acétonitrile;
- l'amino-2-(benzofurannyl-3)-3-propionitrile, à partir du (benzofurannyl-3)acétonitrile;
- l'amino-2-(indolyl-3)-3-propionitrile, à partir de l'(indolyl-3)acétonitrile.

B. Deuxième méthode

10 millimoles (1,33 g) de phénoxyacétonitrile sont mises en solution dans 10 ml de toluène anhydre à 0° C, sous atmosphère d'argon. Une solution de vitride ($NaH_2Al[OCH_2-CH_2-OCH_3]_2$) à 70% dans le toluène (1,4 ml soit 0,5 éq.) dans 3 ml de toluène anhydre est ajoutée, goutte à goutte, à cette température. L'agitation est maintenue durant 1 heure à 0° C, puis 2 ml (1,5 éq.) de cyanure de triméthylsilyle sont ajoutés. Le milieu réactionnel est maintenu sous agitation pendant 3 heures à température ambiante, puis hydrolysé de façon classique. Après purification par extraction acide-base, on obtient 0,300 g d'amino-2-phénoxy-3-propionitrile avec un rendement de 20%.

EXEMPLE 2

Préparation de l'amino-2-phényl-2-acétonitrile

A. Première méthode

a) 10 millimoles (1,031 g) de benzonitrile sont mises en solution dans 10 ml de toluène anhydre, à 0° C, sous atmosphère d'argon. 15 millimoles d'une solution toluénique 1,5M d'hydrure de diisobutylaluminium (10 ml) sont ajoutées, goutte à goutte, à cette température. Après avoir été maintenu à 0° C pendant 1 heure, le mélange réactionnel est additionné de 2 ml (soit 1,5 éq.) de cyanure de triméthylsilyle. L'agitation est maintenue pendant 3 heures à température ambiante; le milieu est alors hydrolysé avec 10 ml de méthanol puis par du sulfate de sodium en bouillie. Après purification de l'α-aminonitrile par extraction acide-base, on obtient 0,820 g d'amino-2-phényl-2-acétonitrile, avec un rendement de 62%.

b) En opérant de la même façon, mais en utilisant comme agent réducteur 0,5 équivalent d'une solution toluénique de vitride à 70% (1,4 ml) diluée dans 5 ml de toluène anhydre (en remplacement de la solution toluénique d'hydrure de diisobutylaluminium), on obtient 0,760 g d'amino-2-phényl-2-acétonitrile; Rendement: 57,5%.

B. Deuxième méthode

a) 20 millimoles (2,06 g) de benzonitrile sont mises en solution dans 20 ml de toluène anhydre, à 0° C, sous atmosphère d'argon. 30 millimoles d'une solution toluénique 1,5M d'hydrure de diisobutylaluminium (20 ml) sont ajoutées, goutte à goutte, à cette température. Après maintien à 0° C pendant 1 heure, le milieu réactionnel est additionné de 50 ml d'une solution normale de cyanure de sodium dans le diméthylformamide. Le milieu est maintenu sous agitation à température ambiante pendant 3 heures, puis hydrolysé avec 20 ml de méthanol et par une bouillie de sulfate de sodium. Après purification de l'α-aminonitrile ainsi formé par extraction acide-base, on obtient 0,350 g d'amino-2-phényl-2-acétonitrile, avec un rendement de 29%.

b) En opérant de la même façon, mais en remplaçant la solution toluénique d'hydrure de diisobutylaluminium par 0,5 équivalent d'une solution toluénique à 70% de vitride (2,8 ml) dans 6 ml de toluène anhydre, on obtient 1 g d'amino-2-phényl-2-acétonitrile. Rendement: 38%.

EXEMPLE 3

Préparation de l'amino-2-phényl-3-propionitrile

A. Première méthode

a) 10 millimoles (1,17 g) de cyanure de benzyle sont mises en solution dans 10 ml de toluène anhydre à 0°C, sous atmosphère d'argon. 15 millimoles d'une solution toluénique 1,5M d'hydrure de diisobutylaluminium (10 ml) sont ajoutées, goutte à goutte, à cette température. Après 1 heure à 0°C, 13,7 ml (soit 1,5 éq.) de cyanure de diéthylaluminium sont ajoutés. Le milieu réactionnel est agité pendant 3 heures avec retour à température ambiante. Il est ensuite hydrolysé par 10 ml de méthanol puis par de la bouillie de sulfate de sodium. Après purification de l'α-aminonitrile obtenu, par chromatographie liquide sur silice, avec élution par acétate d'éthyle/éther de pétrole 1/1, on obtient 0,95 g d'amino-2-phényl-3-propionitrile, sous forme d'une huile orangée. Rendement: 65%.

b) En opérant de façon identique mais en utilisant comme agent de cyanuration le cyanure de triméthylsilyle en remplacement du cyanure de diéthylaluminium, on obtient, après purification par chromatographie liquide sur silice, avec élution par acétate d'éthyle/éther de pétrole 1/1, 0,80 g d'amino-2-phényl-3-propionitrile, sous forme d'une huile orangée, avec un rendement de 55%.

c) En opérant de façon identique mais en utilisant comme agent de cyanuration, le cyanure de tributylstannane ($Bu_3SnCN$), on obtient 0,35 g d'amino-2-phényl-3-propionitrile, sous forme d'une huile orangée, avec un rendement de 24%.

B. Deuxième méthode

1 millimole (0,117 g) de cyanure de benzyle est mise en solution dans 1 ml de toluène anhydre, à 0°C, sous atmosphère d'argon. 1,5 millimoles d'une solution toluénique 1,5M d'hydrure de diisobutylaluminium (1 ml) sont ajoutées, goutte à goutte, à cette température. Après maintien pendant 1 heure à 0°C, une solution d'acide cyanhydrique (en excès) dans le tétrahydrofuranne est ajoutée au milieu réactionnel; lequel est alors maintenu à température ambiante pendant 3 heures, puis est hydrolysé par 4 ml de méthanol et du sulfate de sodium en bouillie. Après une extraction acide-base, on obtient 0,107 g d'amino-2-phényl-3-propionitrile, sous forme d'une huile jaune claire, avec un rendement de 70%.

C. Troisième méthode

a) 10 millimoles (1,17 g) de cyanure de benzyle sont mises en solution dans 10 ml de toluène anhydre, à 0°C, sous atmosphère d'argon. 15 millimoles d'une solution toluénique 1,5M de diisobutylaluminium (10 ml) sont ajoutées, goutte à goutte, à cette température. Le mélange est ensuite maintenu 1 heure à 0°C, puis additionné de 30 ml d'une solution 0,5M (15 millimoles) de cyanure de lithium dans le diméthylformamide. Après 3 heures d'agitation à température ambiante, le milieu est hydrolysé de façon classique. Après une extraction acide-base, on obtient 0,92 g d'amino-2-phényl-3-propionitrile, avec un rendement de 62%.

b) En opérant de façon identique mais en effectuant la cyanuration par addition de 15 ml d'une solution normale de cyanure de sodium dans le diméthylformamide, on obtient 0,871 g d'amino-2-phényl-3-propionitrile, avec un rendement de 60%.

EXEMPLE 4

Préparation de l'amino-2-phényl-4 butène-3-nitrile

a) 10 millimoles (1,29 g) de cinnamonitrile sont mises en solution dans 150 ml de toluène anhydre, à -70°C, sous atmosphère d'argon. 15 millimoles d'une solution toluénique 1,5M d'hydrure de diisobutylaluminium (10 ml) sont ajoutées, goutte à goutte à -70°C. L'agitation à cette température est maintenue pendant 30 minutes; puis 2 ml de cyanure de triméthylsilyle sont ajoutés à -40°C. L'agitation est ensuite maintenue à -40°C pendant 2 heures. Le milieu réactionnel est alors hydrolysé à -20°C par 10 ml de méthanol puis par du sulfate de sodium en bouillie. Le produit obtenu est purifié par chromatographie liquide sur silice, en éluant avec acétate d'éthyle/éther de pétrole 1/1. On obtient alors 0,515 g d'amino-2-phényl-4-butène-3-nitrile purifié. Rendement: 33%.

b) En employant le même mode opératoire mais en remplaçant le cyanure de triméthylsilyle par le

cyanure de diéthylaluminium, on obtient 0,300 g d'amino-2-phényl-4-butène-3-nitrile purifié, fondant à 79° C. Rendement: 20%.

EXEMPLE 5

Préparation de l'amino-2-cyano-4-éthoxy-4-butyronitrile

51,5 millimoles d'éthoxyacrylonitrile sont mises en solution dans 10 ml de toluène anhydre, à -60° C, sous atmosphère d'argon. 77,25 millimoles d'une solution toluénique 1,5M d'hydrure de diisobutylaluminium (51,5 ml) sont ajoutées, goutte à goutte, à -60° C. Après agitation à cette température pendant 45 minutes, 7,7 ml (soit 1,1 éq.) de cyanure de triméthylsilyle sont ajoutés au milieu réactionnel à -50° C. Ce dernier est ensuite maintenu sous agitation à -50° C pendant 3 heures, puis est hydrolysé par du chlorure d'ammonium puis de l'eau. Après purification de l'aminonitrile obtenu par chromatographie liquide sur silice (élution par acétate d'éthyle/éther de pétrole 1/1), on obtient 1,7 g d'amino-2-cyano-4-éthoxy-4-butyronitrile, sous forme d'une huile orangée.

EXEMPLE 6

Préparation de l'amino-2-(méthoxy-5-indolyl-3)-3-propionitrile

a) 21,5 millimoles (4 g) de (méthoxy-5-indolyl-3)acétonitrile sont mises en solution dans 43 ml de toluène anhydre, à 0° C, sous atmosphère d'argon. 32,25 millimoles d'une solution toluénique 1,5M d'hydrure de diisobutylaluminium (21,5 ml) sont ajoutées, goutte à goutte, à cette température. Le mélange est maintenu sous agitation à 0° C pendant 1 heure, avant d'être additionné de 43 ml (soit 1,5 éq.) de cyanure de triméthylsilyle. L'agitation est ensuite maintenue pendant 3 heures à température ambiante puis le milieu est hydrolysé avec 40 ml de méthanol puis par du sulfate de sodium en bouillie. Après purification par extraction acide-base, on obtient 3,28 g d'amino-2-(méthoxy-5-indolyl-3)-3-propionitrile avec un rendement de 71%.

b) En utilisant le même mode opératoire à partir de 27 millimoles (5 g) de (méthoxy-5-indolyl-3)-acétonitrile et en remplaçant le cyanure de triméthylsilyle par un excès d'acide cyanhydrique dans le tétrahydrofuranne, on obtient, après purification par extraction acide-base, 5,7 g d'amino-2-(méthoxy-5-indolyl-3)-3-propionitrile, avec un rendement de 70%.

c) En opérant de manière identique, à partir de 5,4 millimoles (1 g) de (méthoxy-5-indolyl-3)acétonitrile et en remplaçant l'agent de cyanuration par 20 ml d'une solution normale de cyanure de sodium dans le diméthylformamide, on obtient, après purification par extraction acide-base, 0,50 g d'amino-2-(méthoxy-5-indolyl-3)-3-propionitrile, avec un rendement de 43%.

EXEMPLE 7

Préparation de l'amino-2-benzyl-2-phénoxy-3-propionitrile

A. Première méthode

a) 20 millimoles (2,66 g) de phénoxyacétonitrile dans 6 ml d'éther anhydre, on ajoute une solution de 30 millimoles (1,5 éq.) de bromure de benzylmagnésium dans 6 ml d'éther anhydre. Le milieu prend en masse et 10 ml de tétrahydrofuranne y sont ajoutés. Après agitation pendant 2 heures, à température ambiante, le mélange réactionnel est refroidi par un bain de glace, puis additionné de 50 ml d'une solution normale de cyanure de sodium dans le diméthylformamide. Après 2 heures d'agitation à température ambiante, le milieu est hydrolysé par 10 ml de méthanol puis par du sulfate de sodium en bouillie. On obtient 3,45 g de produit brut qui, purifiés par chromatographie liquide sur silice, avec élution par acétate d'éthyle/éther de pétrole 1/1, donnent 0,3 g d'amino-2-benzyl-2-phénoxy-3-propionitrile. Rendement: 6,3%.

B. Deuxième méthode

Dans un ballon à trois tubulures pourvu d'un septum et d'une agitation magnétique, on introduit 0,0069 M de phénoxy-acétonitrile dans 20 ml d'éther anhydre. On refroidit dans un bain de glace puis on ajoute sous argon une solution de bromure de benzylmagnésium dans l'éther (0,00759 M soit 1,1 éq.). On laisse

revenir à température ambiante le mélange réactionnel puis on maintient sous agitation pendant 1 heure. Le mélange prend une coloration rouge puis se prend en masse. On dilue alors avec 5 ml de tétrahydrofuranne sec.

On refroidit le ballon dans un mélange réfrigérant puis ajoute goutte à goutte 1,2 ml de cyanure de triméthylsilyle (0,009 M soit 1,3 éq.). Le mélange se prend en masse. On ajoute ensuite 10 ml de toluène sec. Le mélange reste hétérogène. On laisse revenir à température ambiante et laisse sous agitation pendant 2 heures.

On ajoute quelques gouttes de méthanol puis détruit l'excès de réactif par une bouillie de sulfate de sodium. On filtre puis concentre le filtrat. Le résidu sec est repris par de l'éther isopropylique. On obtient une précipitation d'un produit beige (poids 0,4 g) fondant à 118°.

On purifie par CCM en utilisant un solvant formé d'éther de pétrole-acétate d'éthyle (70/30), le rendement est de 23%.

Le spectre IR est conforme à la structure. Dosage de la fonction amine par l'acide perchlorique, T = 97,45%. Dosage des azotes: 101,3%.

RMN du proton dans $CDCl_3$ (référence TMS $\delta$ = 0)
$\delta$ 1,96 - 2H ($NH_2$)
2,99 - dd 2H ($CH_2$)
3,96 - dd 2H ($CH_2O$)
6,85-7,4 10H aromatiques.
Ce produit est nouveau.

Les $\alpha$-aminopropionitriles ainsi obtenus peuvent être cyclisés en composés hétérocycliques. Ils peuvent également être réduits en $\beta$-diamines ou soumis à une nouvelle opération d'amino-cyanuration.

## Revendications

**1.** Un procédé d'obtention d'$\alpha$-aminonitriles de formule générale I

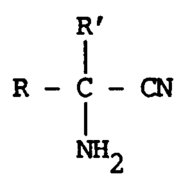

(I)

dans laquelle
R est un radical aliphatique, aromatique ou cyclanique
et R' représente un atome d'hydrogène, un radical alcoyle ou aryle
caractérisé en ce que l'on soumet un nitrile aliphatique, aromatique ou cyclanique de formule générale II

R - CN (II)

dans laquelle
R à la définition énoncée ci-dessus
à l'action d'un agent réducteur métallique choisi dans le groupe constitué par un hydrure d'Aluminium, un sel organomagnésien de formule R'MgX, un sel organozincique de formule R'Zn Hal, un sel organocadmien et un sel organocuprique pour former une imine métallique de formule générale III

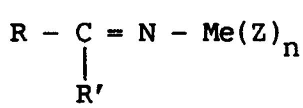

(III)

dans laquelle
R et R' sont tels que précédemment définis
Me est un atome de métal choisi parmi l'Aluminium, le Magnésium, le Zinc, le Cadmium et le Cuivre

Z est un radical alcoyle inférieur ou un atome d'halogène,
et n est un nombre entier égal à la valence du métal diminué de un et l'on soumet celle-ci à l'action d'un agent de cyanuration choisi dans le groupe constitué par le cyanure de trimethylsilyle, le cyanure de diéthylaluminium, le cyanure de tributylstannane, les cyanoborohydrures de métal alcalin, les cyanoborohydrures de métal alcalino-terreux, les cyanures de métal alcalin et l'acide cyanhydrique pour obtenir un α-amino nitrile de formule générale I.

2. Un procédé selon la revendication 1°, dans lequel l'agent réducteur métallique est un hydrure d'aluminium ayant au moins un atome d'hydrogène disponible.

3. Un procédé selon l'une des revendications 1 ou 2, dans lequel l'agent réducteur métallique est l'hydrure de diisobutylaluminium, l'hydrure de diterbutoxyaluminium ou le di(méthoxyéthoxy) aluminohydrure de sodium.

4. Un procédé selon la revendication 1°, dans lequel l'agent réducteur métallique est un agent alcoylant ou arylant choisi parmi le groupe constitué par les sels d'alcoyl ou d'arylmagnésium, les sels d'alcoyl- ou d'arylcadmium, les sels d'alcoyl- ou d'arylzinc et les sels d'alcoyl- ou d'arylcuivre.

5. Un procédé selon la revendication 1°, dans lequel les deux réactions sont effectuées successivement dans le même milieu réactionnel.

6. Un procédé selon l'une des revendications 1 à 5°, dans lequel les deux réactions sont effectuées à basse température comprise entre -30° et +10°C et sous atmosphère de gaz inerte.

7. Un procédé selon l'une des revendications 1 à 6° dans lequel au départ du benzonitrile, on obtient le 2-amino 3-phényl propionitrile.

8. Un procédé selon l'une des revendications 1 à 6°, dans lequel au départ du (5-méthoxy indolyl-3) acétonitrile, on obtient le 2-amino 3-(5-méthoxy indolyl-3) propionitrile.

9. Un procédé d'obtention des α-amino acides optiquement actifs ou racémiques qui consiste à soumettre un α-amino nitrile de formlule générale I obtenu selon l'une des revendications 1 à 8° à une hydrolyse chimique ou enzymatique.

## Claims

1. A process for producing α-amino nitriles of general formula I

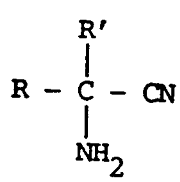

(I)

wherein R is an aliphatic, aromatic or cyclanic radical
and R' is a hydrogen atom, an alkyl radical or an aryl radical
which consists in that an aliphatic, aromatic or cyclanic nitrile of general formula II

R - C N (II)

in which R has the above-given definition
is submitted to the action of a metallic reducing agent selected from the group consisting of an Aluminium hydride, an organomagnesium salt of the formula R'MgX, an organozinc salt of the formula R'ZnHal, an organocadmium salt and an organocopper salt, to produce a metallic imine of general formula III

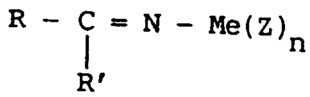

(III)

wherein R and R' are such as previously defined
Me is a metallic atom selected among Aluminium, Magnesium, Zinc, Cadmium and Copper
Z is a Lower alkyl radical or a halogen atom
and n is an integer equal to the valence of the metal less one
and the latter is submitted to the action of a cyanidating agent selected from the group consisting of trimethylsilyl cyanide, diethylaluminium cyanide, tributylstin cyanide, alkalimetal cyanoborohydrides, earth alkaline cyanoborohydrides, alkali metal cyanides and hydrocyanic acid, to obtain an $\alpha$-amino nitrile of general formula I.

**2.** A process according to claim 1 wherein the metallic reducing agent is an aluminium hydride having at least one hydrogen atom available.

**3.** A process according to any of claims 1 and 2 wherein the metallic reducing agent is diisobutylaluminium hydride, ditertbutoxyaluminium hydride or sodium di(methoxyethoxy) aluminium hydride.

**4.** A process according to claim 1 wherein the metallic reducing agent is an alkylating or arylating agent selected from the group consisting of alcoylmagnesium salts, arylmagnesium salts, alcoylcadmium salts, arylcadmium salts, alkylzinc salts, arylzinc salts, and the alkyl or arylcopper salts.

**5.** A process according to claim 1 wherein both reactions are successively performed in the same reaction medium.

**6.** A process according to any of claims 1 to 5 wherein both reactions are performed at low temperatures between - 30$^\circ$ and + 10$^\circ$c and under inert gas atmospher.

**7.** A process according to any of claims 1 to 6 wherein starting from benzonitrile, 2-amino 3-phenyl propionitrile is produced.

**8.** A process according to any of claims 1 to 6 wherein starting from (5-methoxy indolyl-3) acetonitrile, 2-amino 3-(5-methoxy indolyl-3) propionitrile is produced.

**9.** A process for producing optically active or racemic $\alpha$-amino acids which consist in submitting an $\alpha$-amino nitrile of general formula I obtained according to any of claims 1 to 8, to a chemical or enzymatic hydrolysis.

**Patentansprüche**

**1.** Verfahren zur Herstellung von $\alpha$-Amino nitrilen der allgemeinen Formel I

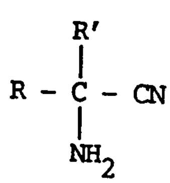

(I)

worin R eine aliphatische, aromatische oder cyclanische Rest
und R' ein Wasserstoff atom, eine Alkyl oder arylgruppe ist
dadurch gekennzeichnet ist dass man ein aliphatisches, aromatisches oder cyclanisches Nitril der allgemeinen Formel II

R - CN (II)

worin R die oben angegebene Bedeutungen hat, der Einwirkung eines reduzierendes metallischen Mittels aus der Gruppe aus Aluminiumhydrid, einen Organomagnesium Salz der Formel R'MgX, einen Organozink Salz der Formel R'ZnHal, einen Organocadmium Salz und einen Organokupfer Salz entstehende unterwirft, um eine metallisches Imin der allgemeinen Formel III

$$R-\underset{\underset{R'}{|}}{C}=N-Me(Z)_n \qquad (III)$$

worin R und R' die selbe Bedeutungen wie vorher, haben
Me eine Metal Atom unter Aluminium, Magnesium, Zink, Cadmium und Kupfer gewählt ist
Z eine niedrig Alkyl Rest oder eine Halogen ist
und n ein Zahl der gleich der Valenz des Metalls minder ein ist, bedeutet
zu bilden,
und dieses, die Einwirkung eines Zyanidierunsmittels aus der Gruppe aus Trimethylsilyl Cyanid, Diaethylaluminium Cyanid, Tributylzinn, Alkalimetall Cyanoborhydrid, Erdalkalimetall Cyanborhydrid, Alkali metall Cyanid und Cyanwasserstoff gewahlt ist
unterworfen wird um eine $\alpha$-Aminonitril der allgemeinen Formel I zuruckzugewinnen.

2. Verfahren nach Anspruch 1, worin der metallische reduzierende Mittel ein Aluminium Hydrid ist, der mindenstens eine zugängige Wasserstoff atom besitzt.

3. Verfahren nach einer der Ansprüche 1 oder 2, worin der metallische reduzierende Mittel Diisobutyl Aluminium Hydrid, Ditertbutoxyaluminium Hydrid oder Natrium di(Methoxyethoxy) Aluminium Hydrid ist.

4. Verfahren nach Anspruch 1, worin der metallische reduzierende Metall eine alkylierende, oder eine arylierende-Mittel ist der, aus der gruppe aus Alkyl - oder Aryl Magnesium Salze, Alkyl - oder Arylcadmium Salze, Alkyl - oder Arylzink Salze und Alkyl - oder Arylkupfer Salze entstehende Gruppe gewählt ist.

5. Verfahren nach Ansprüche 1 worin die beide Reaktionen in dem selben Reaktionsmedium nacheinander ausgeführt sind.

6. Verfahren nach einer der Ansprüche 1 bis 5 worin die beide Reaktionen bei niedrigen Temperaturen zwischen - 30 und + 10 °C und unter inert Gas Atmosphar ausgeführt sind.

7. Verfahren nach einer der Ansprüche 1 bis 6 worin ausgehend von Benzonitril, 2-Amino 3-phenylpropionitril gewonnen wird.

8. Verfahren nach einer der Ansprüche 1 bis 6 worin ausgehend von (5-Methoxy indolyl-3) acetonitril, 2-Amino 3-(5-methoxyindolyl-3) propionitril gewonnen wird.

9. Verfahren zur Herstellung von optisch-aktiven oder razemischen $\alpha$-Amino säuren der nach dem Verfahren der Ansprüche 1 bis 8 hergestellt geworden sind, ein chemische oder enzymatische Hydrolysis unterwirft.